# EUROPEAN PATENT APPLICATION

(11) **EP 3 824 841 A1**
(43) Date of publication of application: **26.05.2021**
(21) Application number: 20187950.9
(22) Date of filing: 27.07.2020
(51) Int. Cl.: A61B 90/98

(54) **METHOD FOR IDENTIFYING AND TRACKING MEDICAL INSTRUMENTS**

(30) Priority: 19.11.2019 BR 102019024415
(71) Applicant: Seweck 4 Administração De Bens Próprios S/A, 20040-001 Rio De Janeiro (BR)
(72) Inventor: MARQUES DOS SANTOS FILHO, CLAUDIONOR, 22775-045 Rio de Janeiro (BR)
(74) Representative: Ferreira, Maria Silvina

(57) **Abstract**

The present patent application, which belongs to the field of logistics, conceives and describes a method for identifying and tracking medical, dental and similar instruments or utensils, by incorporating a RFID (radio frequency identification) type tag in the structure thereof, associated with a digital database, where all the information referring to the instruments is contained, which is integrated with computer equipment interlinked to RFID readers, query terminals and similar, more precisely the method is distinguished by creating an indented slot (1) with a shape and size that is compatible with the RFID-type tag (2) in the structure of the instrument to be identified, depositing a layer of the adhesive element at the bottom of said indented slot (1) and overlaying the RFID-type tag (2), so that its upper face is flush with the surface of the instrument (3) and finally creating a digital database with the information of the instruments (3) referring to the identifications of the respective RFID tag (2).

## Description

### Technical field

The present application describes a method for identifying and tracking medical, dental, and similar utensils or instruments.

### Background

As is known, particularly by those skilled in the art, currently there are two ways of attaching "RFID" tags on medical utensils and instruments, the first way is to encapsulate the RFID tag in medical grade plastic, compatible with sterilization, coupled to a metal base also of medical grade, which is described in patent document BR 102017008602, entitled "SYSTEM AND METHOD FOR ELECTRONIC GOVERNANCE OF SURGICAL UNIT BY AUTOMATING, IDENTIFIYING, AUTHENTICATING AND TRACING ITEMS AND PEOPLE BASED ON REAL-TIME LOCATING SYSTEM WITH SAFE TECHNOLOGY", which discloses systems for automating, identifying, authenticating and tracing items and people based on a real-time locating system with safe technology for the hospital environment. Where equipment, accessories, "RFID" tags were developed so that, coupled to the items, people and locations, monitor usual procedures of the institution, allow real-time registration and monitoring, automation and visibility of the processes, automatic alerts as regards non-conformities, providing patients with safety, electronic governance with reliable data and reduction of the operation costs and health costs.

The second way of fixation is for the RFID tag to be covered with a glue or an adhesive, being completely covered with the adhesive so that it is completely isolated from the surrounding environment.

### Negative points of the state of the art

The greatest inconvenience in these two ways of incorporating the RFID tag in the instrument lies in the fact that there may be accumulation of microorganisms on the surface of the adhesives, which might increase the risk to the patients, when the equipment is used.

Furthermore, the fact that the RFID tag is embossed, might create difficulties in the handling of the respective instrument and might also cause cuts in the gloves of the users, increasing the tendency to contaminations.

Finally, the way in which the RFID tags are currently incorporated in the utensils and medical instruments, leads to uncertainties as regards the handling of the equipment by the doctors, dentists, etc.

### Proposed solution

Thinking about these inconveniences, after numerous research and studies, the inventor, a person connected to the field, created and developed the object of the present patent, idealizing and describing a "METHOD FOR IDENTIFYING AND TRACKING MEDICAL INSTRUMENTS" where not only the mechanical and functional qualities were considered in the manufacturing project, but also the form, disposition and placing of the parts and components that, correctly positioned, brought an increase in the efficiency without any onus.

Its innovative concept allows to obtain an excellent level of functionality, offering a method for identifying and tracking medical instruments, that is extremely reliable, having been created, mainly, to solve the inconveniences caused by the ways in which the RFID tags are currently incorporated in the medical, dental and similar instruments.

The object of the present patent application presents and describes a method for identifying medical, dental and similar instruments, by incorporating a RFID (radio frequency identification) type tag in the structure thereof, a digital database integrated with a set of computer equipment interlinked to RFID readers, query terminals and similar, the novelty of which comprises creating an indented slot in the body of the utensil or instrument and placing the RFID in the same, where the adhesive material is located below the RFID tag.

In this manner, the present invention allows to trace medical and dental equipment without interfering in the way they are handled and avoiding contamination.

Thus, it must be understood that the method in question is extremely simple to carry out, however excellent practical and functional results are obtained, offering an innovative solution to the tracking of medical and similar instruments.

The RFID tags incorporated in medical and dental instruments according to the method being presented, do not interfere with the handling of the respective instruments, and do not lead to contamination risks either.

### Summary

The present application describes a method for identifying and tracking medical, dental and similar utensils or instruments, by incorporating a RFID (radio frequency identification) type tag in the structure thereof, associated with a digital database, where all the information referring to the instruments is contained, which is integrated with computer equipment interlinked to RFID readers, query terminals and similar, characterized by initially creating an indented slot with a shape and size that is compatible with the RFID-type tag in the structure of the instrument, also predicting the volume of the adhesive element that attaches the tag to the structure of said instrument; then, a layer of the adhesive element is placed in the indented slot and the RFID-type tag is overlaid, so that the upper face is flush with the surface of the respective instrument and finally a digital database is created with the information on the instruments referring to the identification of the respective RFID tag.

### Brief description

The object of the present patent application is a practical and innovative method for identifying and tracking medical instruments, belonging to the field of logistics, for use more precisely in identifying and tracking hospital, laboratory, ambulatory and similar utensils, such as surgical instruments, dental instruments, etc.

It is a method that identifies and tracks the medical and dental instruments by electronic means, where an RFID (radio frequency identification) tag with specific information is incorporated in each medical utensil or instrument attributing to it a unique identification, the reading of which may be carried out in a simple and practical manner by means of a proper equipment for the RFID reading. This procedure has the purpose of tracking equipment, utensils, instruments, etc. and allows a more efficient control over the location of same and the number of times in which they were reprocessed.

Thus, in the present application a method especially projected and developed for obtaining enormous practicality is presented and which brings great advantages in the use thereof.

It is a further objective of the present application to present a method for identifying and tracking medical and dental instruments with low costs relating to the use thereof, while allied to the safety and practical requirements, thus offering to the consumer public a method that offers numerous possibilities and benefits to the users thereof, making it a highly acceptable method for the medical, dental and similar sectors.

Since these are vitally important articles for the operations and safety of the patients in hospital, dental institutions, etc., said medical and dental instruments are strictly tracked and monitored.

For this purpose, the most diverse techniques are applied, such as barcodes, "QR" codes, color codes, etc., which are printed on the instrument, for optical scanning.

More recently, the tracking by electronic means has been incorporated in the health sector, with the incorporation of the RFID (radio frequency identification) tag in medical and dental instruments.

Said RFID is an identification technology that uses a radiofrequency to capture data and allows that a transponder be read without the need of a direct visual field and even through objects consisting of the most diverse materials, such as wood, plastic, paper, etc.

For the RFID communication to occur, two components are basically necessary: a reader with antenna and the transponder or RF Tag, which may also be a recorder, in case it is necessary to inscribe new data in the transponder chip. There are passive, semi-passive, and active transponders. When a passive transponder gets close to the reader, the radiofrequency field of the reader is activated and feeds the transponder, which only then can transmit data from its memory to the reader and vice-versa.

### Brief description of the drawings

For an easier understanding of the present application there are figures attached which represent embodiments that, however, do not intend to limit the technology herein disclosed.

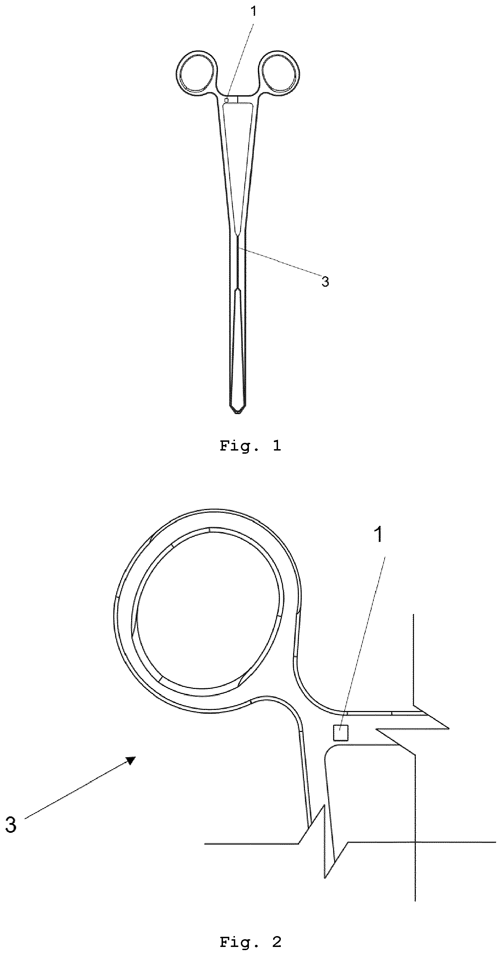

Figure 1 illustrates a front view of a medical instrument (3) with the indented slot (1) for incorporating the RFID tag (2), which are integral parts of the method for identifying and tracking medical instruments (3).
Figure 2 illustrates a front view of the detail of the portion where the slot (2) is in the medical instrument (3) of Figure 1.

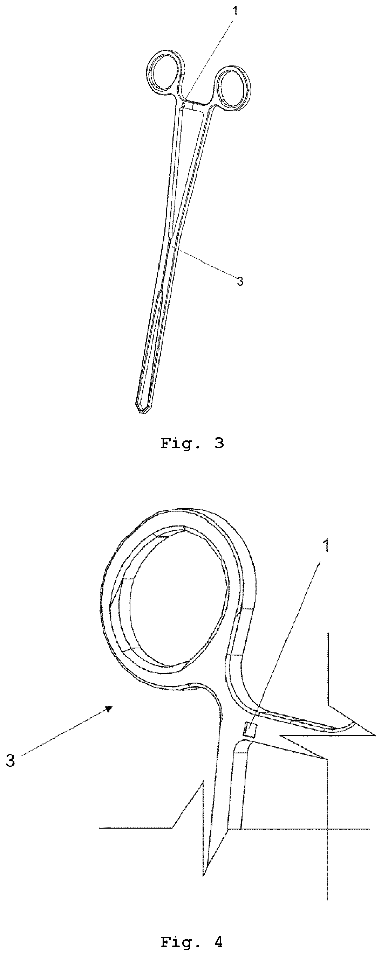

Figure 3 illustrates an isometric view of a medical instrument (3) with the indented slot (1), for incorporating the RFID tag (2), where the depth of same may be seen.
Figure 4 illustrates an isometric view of the detail of the portion where the slot (1) is in the medical instrument (3) of Figure 3.

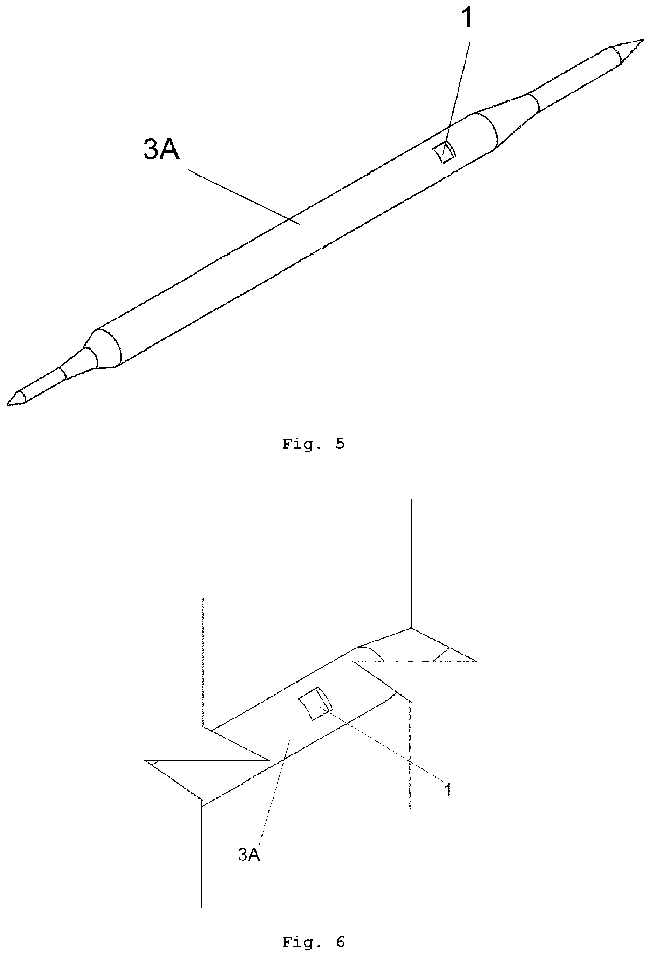

Figure 5 illustrates an isometric view of a dental instrument (3A), with the indented slot (1), representing the replicability of the innovation in different instruments.
Figure 6 illustrates an isometric view of the detail of the portion where the slot (1) in the dental instrument (3A) of Figure 5 is located.

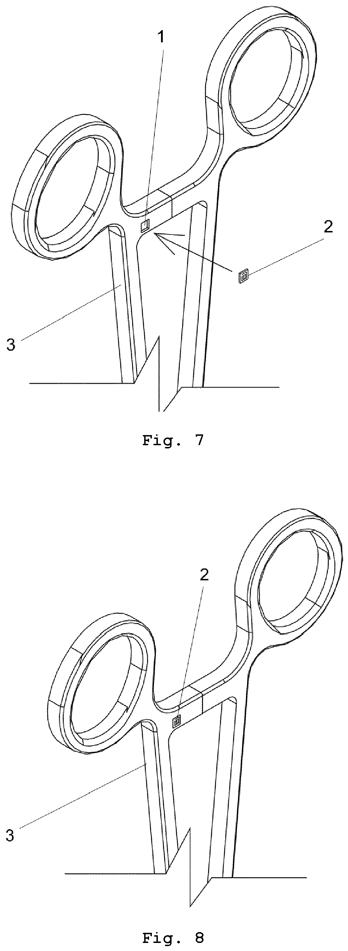

Figure 7 illustrates an isometric view of an RFID tag (2) being installed in a slot (1) executed in the structure of a medical instrument (3).
Figure 8 illustrates an isometric view of an instrument (3) with the RFID tag (2) installed in the indented slot (1), representing one embodiment of the invention.

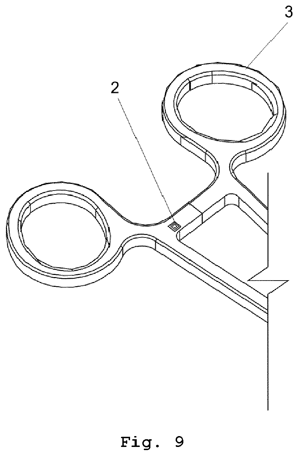

Figure 9 illustrates a perspective view of an instrument (3) with the RFID tag (2) coupled in the indented slot (1), where the absence of embossing in the same can be observed.

### Description of embodiments

Referring to the figures, some embodiments are now described in a more detailed manner, which do not intend, however, to limit the scope of the present application.

In harmony with the above-listed figures, the "METHOD FOR IDENTIFIYING AND TRACKING MEDICAL INSTRUMENTS", object of the present patent application, comprises a method for identifying medical (3), dental (3A), and similar instruments, by incorporating a RFID (radio frequency identification) type tag in the structure thereof, a digital database integrated with a set of computer equipment interlinked to RFID readers, query terminals and similar.

For this purpose, an RFID-type tag (2) is used with specific and pertinent information, being incorporated in a medical (3) or dental (3A) instrument and the information contained in the RFID (2) becomes the identity of the respective instrument.

All information pertaining to the instrument (3) is inserted in a digital database, the contents of which are updated in each operation and processing executed with the instrument (3) .

In this manner, consulting the database it is possible to track any instrument (3) and raise all the history thereof.

It is also possible to carry out the reading of the respective RFID and to obtain the information on the respective instrument (3) contained in the database.

More particularly, the method comprises creating an indented slot (1) with a shape and size that is compatible with the RFID-type tag (2) in the structure of the instrument (3), also predicting the volume of the adhesive element that attaches the tag to the structure of the instrument (3).

Then, a layer of adhesive element is deposited at the bottom of the indented slot (1) and the RFID-type tag (2) is overlaid on the same, so that its upper face is flush with the surface of the respective instrument (3).

Finally, a digital database is created with the information of the instruments (3) which must be referenced to the identifications of the respective RFID tags (2).

Said indented slot (1) can be executed by means of a laser engraving equipment or machine tool, such as, for example, a CNC milling cutter.

The invention has a small separation between the RFID tag (2) and the indented slot (1), which facilitates the placing of the material used to attach said tag.

In this manner, the present invention allows the tracking of medical, dental, and similar equipment without interfering in the way they are handled and, especially, avoiding contamination.

It is certain that when the present invention is put into practice, alterations may be introduced referring to certain details of shape and construction, without implying that this departs from the main principles which are clearly substantiated in the set of claims, it being understood that the terminology used had the purpose of description and not of limitation.

The present description is not, naturally, in any way restricted to the embodiments presented herein and a person with average skills in the field can predict many possibilities for modifying the same, without departing from the general idea, such as defined in the claims. The preferred embodiments described above are obviously interchangeable among themselves. The following claims further define preferred embodiments.

## Claims

1. Method for identifying and tracking medical, dental and similar utensils or instruments, by incorporating a RFID (radio frequency identification) type tag in the structure thereof, associated with a digital database, where all information referring to the instruments is contained, which is integrated with computer equipment interlinked to RFID readers, query terminals and similar, **characterized by**
initially creating an indented slot (1) with a shape and size that is compatible with the RFID-type tag (2) in the structure of the instrument (3), also predicting the volume of the adhesive element that attaches the tag to the structure of said instrument (3);
then, a layer of the adhesive element is deposited on the indented slot (1) and the RFID-type tag (2) is overlaid on the same, so that the upper face thereof is flush with the surface of the respective instrument (3) and finally a digital database is created with the information of the instruments (3) referring to the identifications of the respective RFID tag (2).
